# EUROPEAN PATENT APPLICATION

(11) **EP 4 528 304 A1**
(43) Date of publication of application: **26.03.2025**
(21) Application number: 23199347.8
(22) Date of filing: 25.09.2023
(51) Int. Cl.: G01R 33/54, G01R 33/565, G01R 33/48

(54) **DENTAL MAGNETIC RESONANCE IMAGING IN THE PRESENCE OF METALLIC MATERIALS**

(71) Applicant: Siemens Healthineers AG, 91301 Forchheim (DE); Sirona Dental Systems GmbH, 64625 Bensheim (DE); Dentsply Sirona Inc., York, PA 17401 (US)
(72) Inventor: Biber, Stephan, 91056 Erlangen (DE); Burzan, Kim, 69488 Birkenau (DE); Greiser, Andreas, 91054 Erlangen (DE); Hayes, Carmel, 80634 München (DE); Krüger, Gunnar, 4102 Binningen (CH); Lauer, Lars, 91077 Neunkirchen (DE); Ulrici, Johannes, 64285 Darmstadt (DE)
(74) Representative: Siemens Healthineers Patent Attorneys

(57) **Abstract**

The invention relates to a method for carrying out a dental magnetic resonance imaging examination of a field-of-view (22) within a subject, the field-of-view (22) including a dental area of the subject (20), the method comprising the steps of (a) determining whether a metallic object is present within or near the field-of-view, (b) initiating a standard examination workflow (82) if it has been determined that no metallic object is present within or near the field-of-view, and initiating a modified examination workflow (80, 54, 60, 56, 62, 58, 64) that is different from the standard examination workflow if it has been determined that a metallic object is present within or near the field-of-view, (c) carrying out the standard examination workflow (82) or the modified examination workflow depending on the initiation of step (b) .

## Description

The invention concerns a method for carrying out a dental magnetic imaging resonance imaging examination, a non-transient computer-readable medium comprising a computer program and a magnetic resonance imaging system.

Independent of the grammatical term usage, individuals with male, female or other gender identities are included within the term.

There is an increasing interest in applying magnetic resonance imaging (MRI) in dentistry. Dental MRI can furnish important information on inflammatory and other diseases of the jaws, gums and teeth. However, magnetic resonance (MR) examinations are usually seriously affected by the presence of metallic objects in the field-of-view, since metal-related magnetic field distortion may lead to severe degradation of image quality. There are dedicated MR imaging techniques that are configured to reduce metal artefacts. However, the choice of the appropriate method may depend on many parameters, e.g., the type of the metallic object, as well as its position and size. In dental MRI, the situation is aggravated by the fact that the prevalence of metallic crowns, fillings and implants in the population is very high. Moreover, dentists expect MRI to deliver images of instantaneous diagnostic value, in order for it to be a valuable technique.

In order to establish the clinical usage of dental MRI, it is therefore necessary to provide methods to adequately deal with the possible presence of metallic objects in the dental area, without requiring the presence of well-trained MR specialists during the MRI examination.

It is therefore an object of the invention to provide a method and a corresponding MRI system for carrying out a dental magnetic resonance imaging examination, which takes into account the possible existence of metallic objects in the field-of-view efficiently and reliably.

This object is met or exceeded by a method according to Claim 1, a non-transient computer-readable medium according to Claim 13, and a magnetic resonance imaging system according to Claim 14. Further advantages and features are mentioned in the dependent claims, the description and the attached figures.

According to the first aspect, the invention is related to a method for carrying out a dental magnetic resonance imaging examination of a field-of-view within a subject, the field-of-view including a dental area of the subject, with a magnetic resonance imaging system, the method comprising the following steps:
(a) determining whether a metallic object is present within or near the field-of-view,
(b) initiating a standard examination workflow if it has been determined that no metallic object is present within the field-of-view, and initiating a modified examination workflow that is different from the standard examination workflow if it has been determined that a metallic object is present within the field-of-view,
(c) carrying out the standard examination workflow or the modified examination workflow depending on the initiation of step (b).

By means of the invention, it is possible to routinely carry out dental MRI examinations, even by staff that is not specifically trained in MRI, since strategies (preferably automatic or semi-automatic) of how to detect the presence of metallic material in or near the imaging region and how to modify the workflow in order to deal with the situation arising if a metallic material is detected, are presented.

The invention is focused on dental MRI, in which the field-of-view includes and may be limited to a dental area of a subject. The dental area means in particular the area around the jaws, including at least part of the jaw bones, gum and/or teeth, and optionally the throat of a subject, wherein the subject may be a human or an animal, preferably a patient undergoing dental diagnostics and possibly therapy. The field-of-view may be a transversal slab through the jaw area of a patient. The field-of-view is preferably limited to the head, but excluding the frontal cortex. The MRI examination may be carried out using a local coil, such as a head coil or a specialized dental coil.

The method of the invention essentially comprises as least three steps: In the first step (a), it is determined whether a metallic object is present within the field-of-view. Several possibilities are disclosed how the presence of a metal implant may be detected before or during the dental MRI examination. The first step may comprise a detection or measurement step, in which information on the dental area of the patient is gathered, which may give an indication on the presence of metal in the field-of-view. Next, the information gathered in the detection step may be analysed, wherein the analysis may yield information on whether a metallic object is present, and optionally further information, such as the exact position, the type of metallic object, its size and optionally a confidence value, which indicates the probability that the determined information on the metallic object is correct. "Position", also termed "location" in this context may mean the relative position in relation to the anatomy of the subject, or the position in relation to the field-of-view, in which the diagnostic scan is to be performed.

Once step (a) has been completed, the invention further provides strategies how to deal with the detected metallic object or objects (if any). In step (b), the standard examination workflow is initiated, if it has been determined that no metallic object is present within the field-of-view. The standard examination workflow preferably means the MR examination that was planned for this patient, without considering whether he or she had metallic implants. A modified examination workflow - different from the standard examination workflow - is initiated if it has been determined that a metallic object is present within the field-of-view. Amongst others, a modified examination workflow may comprise a suitable imaging protocol, which is specifically adapted for imaging near metallic material. Preferably, the modified examination workflow is tailored to the specific findings of step (a), i.e., the modified examination workflow may be adapted not only to the presence or non-presence of metallic objects. It may in addition include features which take into account the nature of the metallic objects, such as its type, material, location as well as the severity of the expected distortions.

Finally, in step (c), the standard or the modified examination workflow is carried out, depending on the initiation of step (b).

The method of the invention is preferably carried out by a control unit. The control unit may, for example, be a processing unit such as a CPU, a GPU, a computer or part of a computer. The computer may be a PC, a server, and/or a console of the magnetic resonance imaging system. The computer may also be a mobile device, such as a laptop, tablet computer or mobile phone. The control unit may in particular comprise a dedicated workflow engine, e.g. comprising a computer program configured to carry out the corresponding method steps. The control unit may be configured to adapt the examination workflow, e.g. by adapting sequence parameters of a diagnostic imaging scan. The control unit may be adapted to adapt and/or change the workflow either automatically and/or to guide a user to adapt and/or change the examination workflow, in particular without the user needing in-depth technical knowledge about the workflow. Hence, the control unit is in particular configured to react to the detection of a metallic object and adapt and/or change the examination workflow accordingly. The modification of the examination workflow may comprise activating metal artefact correction features, in particular by changing sequence parameters of a planned diagnostic imaging scan, replacing scan sequences with specialized metal artefact correction sequences, notifying a user about changes, and/or giving advice to a user about further actions to take. The control unit may be configured to provide a modified examination workflow that in interaction with the user adjusts the workflow step by step, in particular by enabling and/or prompting user input and/or giving information and/or advice to the user.

The method of the invention may be carried out fully automatically. However, it is also possible that one or more steps may require user interaction, for example adjusting the selected field-of-view, assisting the analysis of an image of the jaw region, or, in extreme cases, discontinuing the MRI examination if it is determined in step (a) that the patient cannot receive a dental MRI scan either because of safety reasons, or because it is certain that the images generated will not be sufficient for diagnosis. Thereby, MRI scans which are dangerous or which do not make sense can be avoided.

In the following, various embodiments of step (a) including strategies to detect the presence of metallic objects in or near the imaging region are discussed.

According to an embodiment, step (a) includes accessing a dental record of the subject and determining from the dental record whether a jaw and/or a tooth of the subject includes a metallic object, and optionally determining a position and/or the type of said metallic object. In other words, step (a) may comprise using prior knowledge about the tooth status of the subject. The dental record may be or may be part of a "digital twin" of the subject, in particular a digital patient file. In preferred embodiments, the dental record comprises a dental notation scheme, which is used by dentists by general convention, and which indicates which teeth have fillings, artificial tooth crowns or are replaced by implants. For example, the dental notation scheme may contain the designation of a particular tooth, such as "upper/ left/3" to indicate the upper left canine tooth, followed by a description of the tooth status (implant, crown, filling). Optionally, the material of the filling, crown or implant may be derived from the dental record. Thereby, it is possible to gain information on whether the material is critical for an MR examination, or if the material is unproblematic. In particular, ceramic and plastic materials are uncritical, whereas metals containing ferromagnetic alloying elements such as nickel or cobalt, will probably lead to severe distortions and signal voids in the vicinity of the metallic materials in the mouth. Thus, the dental record may contain information about the position of the metallic object, in particular a designation of the concerned tooth, as well as the type, in particular the material of the metallic object. The position in this context preferably means a designation of a particular tooth according to the dental notation scheme used. According to an embodiment, step (a) will by default comprise an attempt to access a dental record, and will take into account the metallic objects found therein in the further method steps. However, if such dental record is not available, or even if it is available, additional steps may be carried out as part of the step (a) of determining whether a metallic object is present within or near the field-of-view. "near" preferably means near enough that the magnetic resonance image within the field-of-view is affected or may be affected by the metallic object. It may for example mean a distance of up to 3cm, preferably up to 1cm.

According to an embodiment, the step of determining whether a metallic object is present within or near the field-of-view includes accessing an image of the mouth region of the subject, the image having been acquired with a non-MR imaging modality, in particular a photograph, X-ray image, CT-image or three-dimensional image, and analysing the image to determine whether the jaw or any of the teeth of the subject includes a metallic object, and optionally determining the position of said metallic object. The non-MR image may be taken just before the dental MRI examination. However, it may also have been acquired previously, for example during a previous visit to the dentist or dental clinic. The mouth region preferably is or overlaps with the dental area. It may mean the oral cavity.

This embodiment makes use of the fact that metallic objects in the oral cavity are often well-visible on other imaging modalities, in particular on optical photographs. Therefore, it is possible to take a photograph of the open mouth of the subject prior to starting the MR examination. The photograph may then be analysed automatically or semi-automatically or manually, in order to identify visible metallic objects. For example, the photograph may be displayed to the user on a screen, in particular a screen which is part of the user interface of the MR imaging system, and the user is given the possibility to indicate the metal-affected teeth via the user interface, and possible provide additional information e.g. on the type, size and/or material of the metallic object. For example, the user is shown the photograph and asked to use a pointing device to indicate the positions of metallic objects, for example by clicking into the image. In addition or alternatively, the photograph may be analysed for example by a trained algorithm in order to identify metallic objects such as fillings or crowns. Optionally, the trained algorithm will be trained to also identify the type of the metallic object, in particular its material and possibly its size. The results of the algorithm are then presented to the user, who may verify of falsify the algorithm's findings. This method may be applied to an optical photograph, but may also be applied to any other image having been acquired with an imaging modality other than MRI, for example an X-ray image, CT image or a 3-dimensional (3D) image. A 3D image may be an optical 3D image.

By assessing the dental records of the patient and/or by analysing a non-MR image, it is possible to detect metallic objects and to provide an indication to the physician diagnosing the MR images that there may be artefacts present in the MR images due to the presence of metallic material.

According to an embodiment, the step of determining whether a metallic object is present includes carrying out an inductance measurement of the dental area of the subject using an inductance sensor. An inductance sensor, also termed impedance measurement system, preferably consists of a transmit coil and a receive coil, wherein the transmit coil is driven with an alternating current in a frequency range of for example between 1 Hz and 1 MHz, preferably between 10 Hz and 50 kHz, more preferred between 1 and 30 kHz. The transmit coil may also be operated at a single frequency within that range, or at a plurality of frequencies. The alternating current generates a changing magnetic field in the vicinity of the transmit coil, which in turn induces a current in at least one receive coil. When a metallic object is introduced into the vicinity of the transmit and receive coils, the eddy current changes. The change in eddy currents is particularly pronounced if the metallic object is moving with respect to the receive coil. Thus, any change in the field coupling between the transmit and receive coil is found to be potentially a metallic object.

Preferably, the impedance measurement system has a direct data connection to the MRI system with which the dental examination is being carried out, and/or to the digital patient file, so that the results of the impedance measurement can automatically be processed and used for the planning of the dental MR examination, in particular for developing a modified examination workflow.

According to an embodiment, the transmit and receive coils of the impedance measurement system are disposed close to the patient table, and preferably at a fixed position with respect to the MRI system. Thereby, the motion of the patient table can be used to induce eddy currents in the receive coil when operating the inductance sensor. In preferred embodiments, the transmit and receive coils are integrated into the MRI system, for example into the housing of the bore or into the stand of the patient table. The coils may be at the location where the head is placed on the patient bed, or at the entrance of the bore. In useful embodiments, the transmit and receive coils are fixedly disposed under the patient table bedding, such that the patient will be moved with respect to the coils when the patient table is driven into the magnet bore. In another embodiment, the transmit and receive coils form circular rings around the entrance of the bore. Preferably, the transmit and receive coils are arranged at a distance as close as possible to the jaw area of a patient placed upon the patient table, for example at a distance of 5 to 30 cm, preferably, 10 to 20 cm.

In preferred embodiments, the system is first calibrated, wherein the patient table is once moved with respect to the transmit and receive coils with no object or patient placed upon it. Thereby, the signals generated from the motion of the patient table itself, which also contains metallic objects, is recorded as a background signal. This background signal may be used to be subtracted from the actually measured signal during operation of the impedance measurement system. This allows the system to differentiate metallic objects e.g. in the patient from other moving metallic objects of the patient table. In preferred embodiments, the recording of the background signal is made at different speeds of the patient table and/or different acceleration profiles. Thereby, it is possible to generate a calculated background signal for any other combination of patient table speed/acceleration, in order to have an individual calibration signal for any patient table movement. Thus, the system is able to calculate background signals from the actual acceleration and speed profile of each individual patient, which might vary due to different patient weights and tolerances of the patient table control. This will allow the impedance measurement system to detect even small metallic objects, since it will increase the sensitivity.

According to an embodiment, step (a) of determining the presence of a metallic object includes determining a value of confidence that the result of the determination is correct. The value of confidence is in particular a probability that the findings of step (a) are correct. Such confidence values are for example often generated by classifying neural networks, such as a YOLO-network, which may be applied to a non-MR image of the dental area of the patient. Such confidence value is important for the further planning of the dental examination, since, if the confidence value is high, further detection steps may be omitted, and the examination workflow may immediately be adapted to the detected metallic objects, or the standard workflow may be carried out if no metallic object is detected. If, on the other hand, the confidence is low, further detection steps may be carried out. Alternatively, the parameters of the diagnostic MRI scan may be set accordingly, either with more or less metal artefact correction features activated. The information gathered in step (a) may also be absolute information, for example if one knows for sure that the subject has a metallic object or implant, or that it does not.

According to an embodiment, the step of determining whether a metallic object is present includes a magnetic resonance (MR) measurement. Such MR measurement may be carried out in addition to accessing the dental record and/or a non-MR image of the patient. It may also be the only step used for determining whether a metallic object is present. The MR measurement may be an imaging or non-imaging measurement. One or several MR measurements may be carried out in order to detect and optionally further specify any metallic object present in the mouth.

An advantage of the non-imaging MR measurements is that the transmit amplitudes of the RF pulses may be kept low, and the measurement time is very short, e.g., about 1 to 4 transmit pulses, each having a few ms duration each. This ensures that, even for patients having metallic objects such as metal implants, the risk of heating or even sparking is minimized. Imaging solutions, on the other hand, intrinsically give an indication of the position of the metallic object, which may be very useful in the further planning of the dental MR examination.

Metallic objects, especially ferromagnetic and paramagnetic objects, lead to local distortions of the main magnetic field B₀ used for MR measurements. The distortions lead to local shifts in the Larmor frequency in the vicinity of the metallic objects, i.e. the ¹H spins resonate at a slightly different frequency (termed "off-resonant" frequency) than the spins in the rest of the field-of-view. Thus, "off-resonant" means shifted with respect to the Larmor frequency of spins which are not affected by main magnetic field distortions caused by the metallic object(s). The amount of off-resonant signal, and the distance in Hz by which the frequency of the spins near the metallic object is shifted with respect to the other spins in the field-of-view, can give an indication on the size and the material of the metallic object. Therefore, for all MR techniques which measure the Larmor frequency of a voxel or region, or the frequency spectrum of the volume, measuring the exact frequency of the off-resonant components with respect to the reference frequency, i.e., the Larmor frequency of ¹H in an undisturbed field, can also give an indication on the type of material causing the field distortion. This is because different materials have different susceptibilities and therefore also exhibit different Larmor frequencies in their proximity. Therefore, an MR measurement may give an indication on the presence or non-presence of a metallic object, on its material, and also an indication on its position, depending on the spatial resolution used in the measurement.

Several ways of detecting metallic objects using MR measurements are possible in the context of this invention, and are described in the following. For example, the MR measurement may comprise carrying out a spectroscopic measurement to acquire a magnetic resonance spectrum, um, wherein the MR spectrum is analysed to determine the off-resonant signal components as indication of the presence of metallic material. In a preferred embodiment, the spectroscopic or non-imaging measurement is carried out as part of the adjustment of the transmitter (AdjTra). During the transmitter adjustment, a broadband radio frequency (RF) pulse at the Larmor frequency is played, which is usually not slice selective, so that there is an answer from all spins in the sensitive volume of the MRI system. The received spectrum of echoes from all spins in the volume can thus be plotted. Since metallic objects are usually slightly magnetic, the magnetic susceptibility difference between metal and tissue is much larger than the susceptibility difference between air and tissue, which will naturally be found within the sensitive volume. Therefore, the local Larmor frequency (B₀) in the vicinity of a metallic object is expected to be significantly different compared to all other tissues. This will give rise to off-resonant signal components in the AdjTra spectrum. Thus, a comparison of the AdjTra spectrum to a typical spectrum allows the system to identify the presence of metallic objects. In order to get a better information on the rough location of the metallic objects, such as metal implants, the measurement of the spectrum can also be applied in spatially selective way. For example, several (e.g. 2 to 10) different large regions could be selectively excited with broadband RF pulses. Useful regions could be the left body half, right body half, upper jaw, lower jaw, or a combination of left/right and upper/lower. This will allow to get a rough information on the location of possible metallic objects in the jaw without the need to run a full imaging sequence.

According to an embodiment, the MR measurement includes a low-resolution MRI scan, in particular having a spatial resolution which is higher in the occlusal plane than in a direction perpendicular thereto. The "occlusal plane" is an imaginary plane formed by the occlusal surfaces of the teeth when the jaw is closed. The occlusal plane may be derived from a curved surface touching the incisal edges of the incisors and the tips of the occluding surfaces of the posterior teeth. Because the teeth may not be exactly regular, the occlusal plane is preferably an average plane derived from this curved surface.

The low-resolution MRI scan may for example be localizer scan, particularly a gradient-echo based 3D localizer scan (also referred to as scout scan), which is typically performed at the beginning of an MR examinations in order to plan the further diagnostic MRI scans. Alternatively, it may be a 2D localizer scan, which however uses very long voxels preferably in the z-direction and therefore is similar to projection imaging, which allows a very precise determination of the metallic object in the x and y direction, which preferably corresponds to the occlusal plane. For example, the in-plane resolution in the occlusal plane may be 1-20mm. In the z-direction, the voxels may be very long, e.g., 1-10cm long. Thereby, it is possible to cover the complete jaw in a very short time, while at the same time localizing any metallic object precisely in the x-y plane, but without determining the exact position in the z-direction. Finding the implant in the upper or lower jaw is then possible with subsequent techniques, or by simple visual inspection through the operator.

According to an embodiment, the low-resolution magnetic resonance imaging scan is a 3D localizer scan. The low-resolution magnetic resonance imaging scan may have a spatial resolution which is higher in a direction oriented in parallel to the occlusal plane than in a direction perpendicular thereto. Particularly, the low-resolution magnetic resonance imaging scan may have a spatial resolution which is higher in a volume defined by at least a section of a dental arch or sections of both dental arches than in a volume surrounding the section of the dental arch or the sections of both dental arches.

The advantage of using the long voxels is that the method is extremely fast, and very insensitive versus exact patient positioning and the exact anatomy, while delivering good x-y information on the location of the metallic object. Alternatively, a standard 2-dimensional (2D) or 3D localizer scan, preferably gradient-echo based, may be used. Thereby, the first images acquired in a dental MR examination in an individual with known or unknown metallic objects in the oral cavity would provide first visual evidence of the extent of any metal artefact, by showing a signal void close to the metallic object. A "signal void" herein means a region in an MR image in which the signal intensity is low to such a degree that no image information can be obtained from this area. Typically, the signal intensity of pixels/voxels in the signal void may be less than 15%, preferably less than 10%, more preferred less than 5% of the average signal intensity of the body parts or tissue within the field-of-view.

Other MRI sequence types which may be used (e.g. in the low-resolution MRI scan) to identify metallic objects are susceptibility weighted imaging (SWI). SWI is able to identify subtle differences in susceptibility within a tissue by using local face differences, and is therefore sensitive enough to also find metallic objects. For example, gradient-echo based T2*-weighted imaging sequences may be used in SWI. Alternatively, a 2D or 3D map of the Larmor frequencies of individual voxels within the field-of-view will also reveal the presence of metallic objects, by showing voxels having a significantly off-resonant Larmor frequency.

According to an embodiment, the low-resolution MRI scan is configured to have a duration of 0,01 s to 10 s, preferably of 0,1 s to 5 s, more preferably of 1 s to 3 s.

In preferred embodiments, the acquired spectra or images are automatically analysed in order to identify metallic objects, in particular the position and/or type of the metallic objects, and to assess the severity of the metal artefact caused by such metallic object. The analysis may be performed by an algorithm, e.g. a trained algorithm, in particular a neural network that is trained with MR images including metal-induced artefacts, which have been annotated as such by a user. In a preferred embodiment, the dental record including a dental notation scheme which documents the position and type of metal-based accessories in the mouth can be used as prior information to such algorithms. For example, the algorithm will be given the position from the dental notation scheme, and search for metallic object induced artefacts in the acquired MR image. The algorithm may be integrated into a software trained to identify specific teeth and/or specific landmarks in MR images, in particular in the above-described 2D or 3D localizer images. Such software may be trained for example using machine learning techniques, and can similarly be trained to recognize metal-induced artefacts in the image.

In an embodiment, the trained software/algorithm may not only determine those regions in a localizer image which may potentially be affected by metal artefacts, but can also inform the user of such potential artefact regions within the planned image. For example, after the slices or volumes to be scanned in a diagnostic MRI scan according to the standard examination workflow are positioned on the localizer scans, the software may highlight those regions which may potentially be artefact zones, and optionally estimate the impact the artefacts will have on the diagnosis. This feature is highly useful to medical staff with no special MR knowledge, since it will enable them to either reposition the slices or volumes, or at least to know in advance whether the region of interest can at all be diagnosed, or whether it will be too much affected by the metallic object(s).

In an extreme case, the trained software used for analyzing such scout or localizer images may alert a user in the event that the expected metal artefacts are so severe that the respective subject or patient cannot receive a dental MRI scan. Thereby, MR scans which are dangerous or which do not make sense can be avoided.

According to an embodiment, the MR measurement includes a diagnostic MRI scan with a first type of metal artefact correction, wherein the first type may also be no metal artefact correction. In this embodiment, the diagnostic scan, which will be carried out for diagnostic purposes as part of the standard or modified examination workflow, is additionally used to identify the metallic objects and/or evaluate the severity of metal-induced artefacts. The diagnostic MRI preferably has a high resolution having a pixel/voxel size of e.g. 0.5-3mm. The diagnostic MRI scan may be a 3D multispectral MRI (3D-MSI) scan. It may in particular use a normal diagnostic scan sequence used in dental MRI, for example a turbo spin echo sequence, an inversion recovery sequence, a MR sequence using fat suppression, or variants of these. The diagnostic MRI scan may already have some features to reduce metal artefacts, for example a specific type of MR sequence/protocol. Imaging sequences which are specifically adapted for imaging near metallic material use for example one or more of Multi-Slice View Angle Tilting (MS-VAT), SEMAC (Slice Encoding Magnetic Artefact Compensation) or MAVRIC (Multi-Acquisitioned Variable Resonance Image Combination) techniques.

The principle of view-angle tilting (VAT) is that magnetic susceptibility artefacts, such as caused by metallic objects, cause displacements in both the read-out and slice-select directions. Therefore, the in-plane distortions may be reduced by making the off-resonance displacements to cancel each other. This is done by applying an additional VAT gradient during the signal read-out along the slice-selection direction which is equal to the one applied during the slice-selective excitation. These additional read-out gradients result in a "shearing" of the pixels, as if viewing the slice at a slight angle. The VAT method may fully compensate for in-plane pixel shifts in the read-out direction. In order to correct through-plane distortions due to metal, the SEMAC or MAVRIC technique may be employed. The SEMAC technique uses a VAT-correction gradient as explained above, in addition to acquiring additional phase-encoding steps in the slice-select direction in order to minimize through-plane displacements. The additional phase-encoding steps provide information on how the metallic objects have distorted the original slice profile. This information can be used to correct for signals shifted perpendicular to the imaging plane. Finally, MAVRIC relies on a fast 3D spin-echo sequence and uses a narrow band excitation coupled with a multispectral VAT-type read-out.

Further metal artefact correction features which may be used in the first type of metal artefact correction include simply using a higher read-out bandwidth, smaller field-of-view, imaging thinner sections etc. A further MR measurement sequence which is specifically adapted for imaging near metallic material utilizes ultrashort echo time, and which are termed UTE-ZTE sequences.

According to a preferred embodiment, the images acquired with the diagnostic MRI scan using the first type of metal artefact correction (which may be no metal artefact correction) are analysed to determine the presence of metallic objects, and the modified examination workflow includes a diagnostic magnetic resonance imaging scan with a second type of metal artefact correction, wherein the second type of metal artefact correction depends on a result of the analysis of the diagnostic magnetic resonance imaging scan with the first type of metal artefact correction. Especially in the event that the presence of a metal object has been detected, the metal artefact correction may be advantageously improved with respect to the first type of metal artefact correction on the basis of the results of the analysis. In other words, the metal artefact correction is improved during the diagnostic dental MR examination, each diagnostic scan having learned from the previous diagnostic scan. This may be repeated several times, wherein each diagnostic scan is further improved over the previous one. According to preferred embodiments, the type of metal correction is automatically improved based on the analysis. For example, an automatic analysis of the diagnostic MR scan may be carried out, in order to identify any signal voids which indicate the presence of metallic objects. The severity of the artefact is then estimated. Based on the severity of the metal-induced artefact, in particular in relation to the diagnostic aim of the MR scan, the second type of metal artefact correction for the next diagnostic scan is selected. For example, if it is discovered that the metal-induced artefacts are severe, but that further metal artefact reduction features may mitigate the situation, these will be activated in the next diagnostic scan. For example, one of the above-described imaging sequences with metal artefact correction may be used, or a higher read-out bandwidth, a smaller field-of-view, or a thinner section may be imaged. All of this may increase the duration of the examination, but will still be useful if it is a suitable type of metal artefact correction and will result in MR images having diagnostic value.

In the following, the strategies and in particular a modified examination workflow will be described which will deal with the situation arising if metallic objects are present in or near the field-of-view of the planned dental MR examination, according to the various detection methods of step (a).

First of all, if a metallic object is detected in step (a), preferably the modified examination workflow will include carrying out a diagnostic MR scan using an imaging sequence with metal artefact correction, in particular an MS-VAT, SEMAC or UTE imaging sequence, and/or adjusting the parameters of an MRI sequence in order to minimize the artefacts caused by metallic objects. According to a preferred aspect of this embodiment, the specific type of metal artefact correction, and/or the parameters of the selected MRI sequence are set based on the information gathered in step (a). For example, it may be set based on the information whether a metallic material is present at all, and on the confidence value that metallic material is present. It may further be based on the type of metallic material/object. For example, if it is detected that a metallic object, for example a metal implant, is present, but that the type of the metallic material is not ferromagnetic, a lighter form of metal artefact correction may be employed, which does not unnecessarily increase the duration of the diagnostic scan. In a further embodiment, the parameters may be set based on the location of the metallic object, including the spatial position of such object in relation to the region of interest of the diagnostic scan. For example, if a metallic object is detected, but if it is so far away from the region of interest that the region of interest will not be affected, no or only a light metal artefact correction may be required. On the other hand, if the distance is small, the method step (b) of initiating a modified examination workflow may include a detailed analysis including simulations of expected metal artefacts, in order to find out whether a diagnostic is possible at all, and in order to determine a suitable type of metal artefact correction features.

In an example, if the tooth root of a particular tooth X is to be scanned in a dental MRI exam, it may have been detected in step (a) that the tooth next to tooth X has a metallic filling. Therefore, automatically a suitable SEMAC MR measurement sequence is employed in the modified examination workflow, and in particular as the diagnostic imaging scan, in order to generate images of the tooth root of tooth X without artefacts. In addition, it is possible to change around the slice select direction in the diagnostic imaging scan, since metal artefacts may have different defects in different planes.

Generally, a further measure to take when metallic objects are detected, for example from the dental record of the patient, is to employ low-field MR systems, in particular operating at <1.0T, for example 0.55T. Generally, low-field MR reduces the artefacts caused by metallic material. Thereby, it is potentially possible to image near metallic objects without having to use the special MR measurement protocols indicated above, thereby saving examination time. A modified examination workflow may thus include using a low-field MRI system.

A modified examination workflow may also include disqualifying a patient from receiving a dental MRI examination, either because of safety reasons, or because it is certain that the images generated will not have any diagnostic value. This may be the case if the patient has ferromagnetic metal implants, such as nickel cobalt fillings. Thereby, MR scans which are dangerous or which do not make sense may be avoided.

According to an embodiment, the modified examination workflow includes simulating the artefacts caused by the detected metallic object in an MR image. In this embodiment, a numerical simulation to predict artefacts in MR images may be used, given a database containing characteristics of the metallic objects, such as the magnetic susceptibility and the expected orientation of the metallic objects (e.g., fillings, crowns or implants) relative to the main magnetic field. The simulation may be based on the information stored in the "digital twin" of the patient, e.g. in the dental record. Such simulations may be based on a physical model of the metallic object or objects, on the basis of which the susceptibility distribution within the field-of-view and thus the B₀-field distortions may be simulated.

For example, an MR image similar to the planned diagnostic scan of a volunteer and showing the region of interest in the jaw may be used and modified, in order to simulate how the MR image would look like, if the volunteer had the metallic objects which are evident from the dental record of the patient to be imaged. From looking at this simulated MR image, the operator may decide whether the MR examination of the tooth of interest is possible in the first place, in particular if the tooth or area of interest in the jaw is in such proximity to a metal artefact inducing implant or accessory, that a diagnostic MR examination is not possible with currently available techniques.

According to an embodiment, the modified examination workflow includes determining a region within the field-of-view which is affected by the metallic object, and visualizing said affected region in a scout image used for planning the dental magnetic resonance imaging examination, or alerting the user when a diagnostic magnetic resonance imaging scan is planned in said affected region.

In this embodiment, the planning of the diagnostic MR scan can be assisted on the basis of the findings of step (a). In particular, if the position and preferably further features of a detected metallic object is known, in particular its material and size, an algorithm can predict a region, in particular a volume within the field-of-view, which will be affected by the metallic object. This algorithm may be carried out by the control unit as part of the modified examination workflow.

To determine the affected region, the same kind of numerical simulations as described above for the simulation the artefacts caused by the detected metallic object(s) can be used. To delineate the affected region from the results of the simulation, one can for example use a predetermined threshold in the Larmor frequency deviation, and thus determine the volume in which the Larmor frequency is off-resonant to such an extent that severe artefacts up to signal voids are expected. Once this calculation has been performed, the results may be visualized in a scout image or in any other image acquired previously from the dental area of the subject. The affected area may also be visualized for example in an optical image, CT-image, X-ray image or an image taken by any other non-MR imaging modality. The affected area may be visualized either by overlaying a shaded or colored area in the affected region, or by drawing the boundary of the affected region as a line. In other words, the knowledge about an artefact impacted tooth can be translated into a volume around the respective tooth ("radius of uncertainty"), in which the diagnostic confidence is diminished due to the metallic object. By visualizing this volume in the planning data, the operator will be informed which part of the dental anatomy is expected to be impacted by the metallic object. In another embodiment, based on the type or material of the metallic object, a specific, predetermined radius is chosen around the metallic object to define the border of the affected region.

Accordingly, the operator may decide whether diagnostic MR scan can be carried out as planned, or whether it should be modified.

In addition, the user may be alerted when the diagnostic MRI scan is planned in said affected region. This may for example be through a visual or aural signal issued by the MRI system when the diagnostic scan is planned to overlap the affected region.

According to an embodiment, the modified examination workflow includes determining a region within the field-of-view in which a magnetic resonance image includes a signal void caused by the metallic object, and modelling an acquired magnetic resonance image in this region on the basis of a jaw model, or augmenting an acquired magnetic resonance image in this region with image information taken from an image acquired with a non-MR imaging modality of the mouth of the subject. The region in which the MRI image includes a signal void caused by the metallic object may be the same region as said affected region. It may be obtained in the same way, i.e., by simulation or by utilizing a pre-determined radius for a given material or metallic object type. Alternatively, the method may include analyzing an acquired MRI image, which may be a scout image or a full diagnostic MR scan, in order to identify the areas where the MR signal has been spoiled due to metal artefacts. The regions including a signal void may also be the complete regions which are affected by the metallic object or objects, in the sense that any MR signals received from such regions is so distorted that it does not provide useful information. These regions may be replaced by image information obtained by other means. For example, the image information may be obtained by modelling an MR image on the basis of a jaw model. The jaw model may be a generic model, optionally with the metallic object detected in the affected area included into the generic model. The generic jaw model may be positioned within the MR image by using landmarks on the MR image with are still available from the non-affected regions.

Alternatively, the image information may be taken from an image acquired with a non-MR imaging modality of the mouth of the subject, for example an optical image acquired by an intra-oral camera, an X-ray, or CT-image etc.

This embodiment is highly useful, since it is expected to significantly improve the acceptance of dental MRI in the presence of metallic objects in the mouth. This is because the operators will not be confronted with areas of signal voids, which would otherwise severely hamper the readability of the acquired diagnostic MR images. Rather, the operator will always receive an image of the jaw region and will be able to orient himself in the jaw. It may be that the region of interest is not visualized in the MR image, due to signal voids, but at least the operator is presented with alternative image information which may still provide useful information.

According to an embodiment, the modified examination workflow includes alerting a user that a dental magnetic resonance imaging examination will not yield acceptable image quality. The alert is based on the analysis of the findings of step (a) as described herein, for example on the size, number, material and type of detected metallic objects. It may also be based on the determination of a region within the field-of-view in which a magnetic resonance image includes a signal void caused by the metallic object, or which is affected by the metallic object. Thus, the alert can be issued globally, i.e. when an MRI examination is generally not possible. However, it may also be issued locally, in the sense that the user is alerted that an MRI examination will not be possible in a particular region within the field-of-view.

According to a further aspect of the invention, a computer program is provided, which comprises computer-readable instructions which, when carried out on a control unit of an MRI system, will cause the MRI system to carry out the method as described herein.

According to a preferred embodiment, the MRI system comprises and inductance sensor configured for carrying out an inductance measurement of the dental area of the subject. The inductance sensor may have all the features and advantages as described as with respect to the method of carrying out an inductance measurement and vice versa.

In particular, the transmit and receive coils of the inductance sensor may be arranged for example under the top layer or bedding of the patient table, either in the moveable section of the patient table or preferably in the stationary part, so that the patient is moved across the sensitive area of the transmit/receive coils to allow metal detection. The transmit/receive coils are preferably arranged near the top end, where the head of the patient is usually positioned. It may be just outside the bore, in order to possibly alert the operator already before the subject is fully driven into the magnet bore. This has the advantage that the inductance sensor can be used as a safety feature, preventing patients with metal implants to be subjected to the potentially harmful high magnetic field inside the magnet bore. The sensor coils may also be arranged just inside the magnet bore. They may also be integrated into a head coil or specialized dental coil, used for acquiring the RF signal. Finally, the transmit/receive coils may be arranged at the entrance of the bore, either on one side or arranged in a circle around the bore. The inductance sensor may be used before any MR measurements, including localizer images, are started.

The invention shall now be described by means of various exemplary embodiments with reference to the attached drawings. In the drawings:
- Fig. 1: shows a schematic representation of an MRI system according to an embodiment of the invention;
- Fig. 2: shows a schematic cross section through the human head showing a field-of-view including a dental area;
- Fig. 3: is a flow diagram illustrating an embodiment of the inventive method;
- Fig. 4: is an MR image showing a signal void obtained by a metallic object, in particular a metal implant in the jaw;
- Fig. 5: is side view of a patient table including an inductance sensor according to a first embodiment;
- Fig. 6: is side view of a patient table including an inductance sensor according to a second embodiment;
- Fig. 7: is side view of a patient table including an inductance sensor according to a third embodiment.

Similar elements are designated with the same reference signs in the figures.

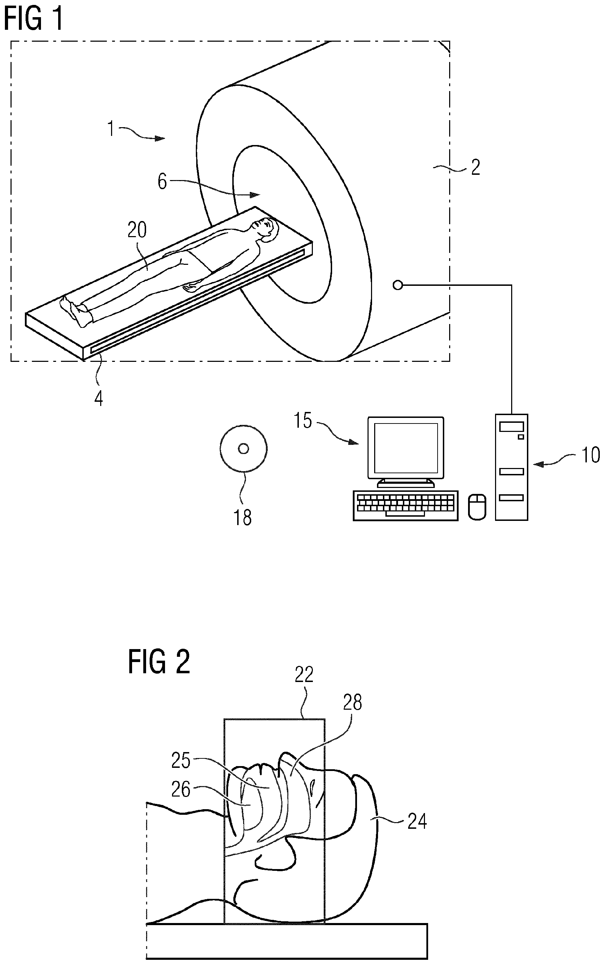

Figure 1 shows an embodiment of a magnetic resonance imaging system 1 according to the invention. The MRI system comprises an MR scanner 2 including a main magnet, and a control unit 10. The control unit 10 is configured to control the MR scanner 2, in particular to control all magnetic resonance protocols carried out on the MRI system. The control unit 10 is further configured to initiate a standard or modified examination workflow including various types of MR scans, including diagnostic scans and localizer scans, with or without metal artefact correction. The control unit 10 in particular initiates a standard examination workflow, which may include scanning the subject with a standard scan protocol if no metallic object is detected, and a modified examination if a metallic object is detected. An operator may interact with the control unit 10 via a user interface 15, which includes a screen, a computer mouse and a keyboard. The method of the invention may be embodied in a computer program stored on a computer-readable medium 18, for example a CD-ROM. The control unit 10 is configured to output information and/or suggestions via the user interface 15, and to receive user input via the user interface 15. A patient 20 is illustrated on a patient bed 4, which may be moved into the bore 6 of the main magnet 2 for MA scanning.

Figure 2 illustrates what is preferably meant by a dental area of a subject. Shown is a head 24 of a patient, with the oral cavity 25 including the tongue 26 and the nasal cavity 28 schematically illustrated. A dental area preferably covers at least part of the oral cavity 25, including the tongue, upper and lower jaw, the hard and soft palate and the larynx. Optionally, the dental area may also cover the nasal cavity 28 and possibly the eustachian tube which connects the throat to the ear. An exemplary field-of-view 22 which may be used for diagnostic dental MR scans is shown, which in this case is a transversely oriented slab covering the jaws and nasal cavity 28.

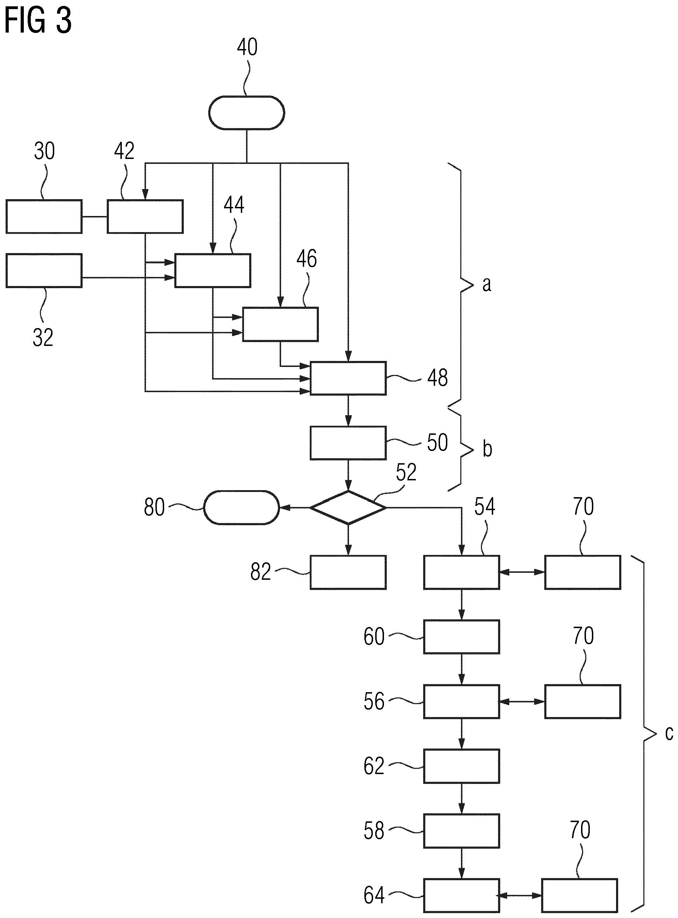

Figure 3 is flow diagram of a method according to an embodiment of the invention. On the left side shown are steps taken and initiated by the control unit 10 and partially executed by the MRI system 1. On the rightmost column are steps requiring user interaction, in particular interaction with an operator of the MRI system 1. The method starts at 40. First, various steps of the metal detection step (a) may be carried out either singly or in combination, preferably one after the other. Each individual step is optional and may be replaced by one or several of the other metal detection steps. For example, in step 42, a dental record 30 of the patient is accessed, and the information taken therefrom is analyzed for the presence and optionally type, material or size of any metallic fillings, implants or crowns. Alternatively or additionally, step 44 of accessing an image 32 of the oral cavity of the subject to be scanned may be carried out. The image may be an optical image taken by an intra-oral camera, a previously acquired X-ray image or a CT-image. It may be analysed in step 44 to identify, localize and preferably characterize any metallic objects contained in the dental area of the subject. Alternatively or additionally, a spectroscopic measurement 46 may be carried out as described herein, for example as part of a transmitter adjust protocol. Therein, off-resonant signal components are identified and analyzed to find out whether they have been caused by metallic objects, and if yes, whether the metallic objects are critical for diagnostic MR scans. In an embodiment, the spectroscopic measurement may be repeated with a rough spatial selection, in order to further characterize the position of a detected metallic object. Further alternatively or additionally, a low-resolution scout MR measurement 48 may be carried out on the field-of-view. Practically, the low-resolution MRI scan requires a duration of 0.01s to 10s, preferably 0.1s to 5s, more preferably 1s to 3s. The low-resolution MRI scan may be a standard localizer scan, in particular a 2D or 3D scan. It may also - in particular depending on whether any metallic objects have been detected in either of the previously described steps 42, 44, 46 - be a specialized metal detection sequence. It may be optimized to detect the presence of metallic objects in the field-of-view. It may for example have a particularly low resolution in the direction perpendicular to the occlusal plane. Alternatively, it may be a regular MR localizer scan.

The results of any metal detection steps 42, 44, 46 and 48 are analyzed in step 50, to determine the further workflow regarding the diagnostic MR scan which is planned for the subject in the field-of-view. Depending on the outcome of the analysis, one of three different workflows are initiated in step 52: if the metal detection step (a) has provided the outcome that the metallic object in the dental area is so big that any MR scan would be dangerous and/or would not be expected to be of any diagnostic value, the operator is alerted that the MR exam should be stopped in step 80. This may be through an optical or acoustic signal. If, on the other hand, no metallic object or only a very small metallic object has been detected, the standard workflow 82 is initiated, which may include various diagnostic MR scans. A diagnostic MR scan is preferably a high-resolution scan intended to give diagnostic information on the dental area of the subject.

If a metallic object has been detected, but it is not expected to have such severe effects on the MR measurement that further scans are excluded, a modified examination workflow 84 is initiated. This workflow may include one or more of the various strategies to minimize the effects of metal implants as described herein. One of the strategies is illustrated in this drawing. This consists of first analyzing the localizer image in step 45. Step 45 may include augmenting the localizer image with models or other non-MR images of the teeth which would be shown at any signal voids caused by metallic objects. It may also include illustrating the region expected to be severely affected by the detected metallic object, and showing such image to the operator 70. The operator may interact with the control unit 10 in order to plan the diagnostic scan on the basis of the visualized information. In the next step 60, a first diagnostic MR scan is carried out. This MR scan 60 may include one or more metal artefact correction features, such as high bandwidth, MS-VAT or SEMAC sequence features, depending on the result of the analysis 52 and the scan planning 54. Once the first diagnostic MR scan 60 has been carried out, the resulting image may be analyzed in step 56. It may be presented to an operator 70 for visual inspection. Preferably automatically, the sequence parameters may hence be modified, depending on the metal artefacts still visible in the image obtained by the first diagnostic MR scan 60. The user may also amend the scan parameters. In a next step 62, a further diagnostic MR scan is carried out, wherein the metal artefact correction features have been modified to take into account the findings of step 56. The image may be further analyzed in step 58, to possibly further improve the metal correction features. On the basis of this analysis, a third diagnostic MR scan 64 may be carried out, and finally presented to the operator 70 as diagnostic image. Evidently the number of diagnostic MR scans 60, 62, 64 may vary according to the needs of the operator and the diagnostic question.

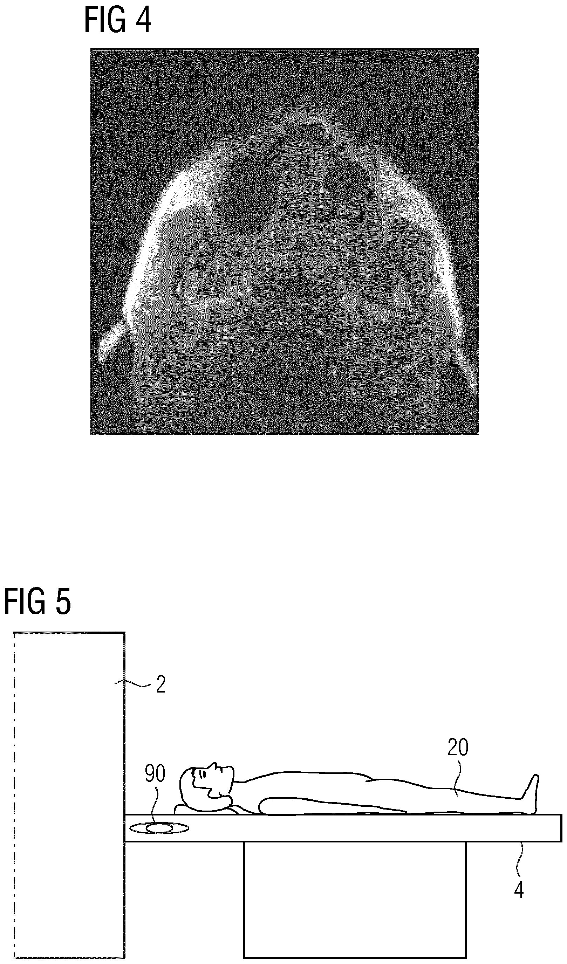

Figure 4 shows an exemplary actual MR image of the jaw of the patient. The signal voids caused by metal implants are clearly visible. On the other hand, the rest of the jaw is still visible at acceptable image quality, thus a diagnostic MR scan is possible.

Figures 5 to 7 illustrate several ways of placing the transmit/receive coils of the inductance sensor. In the embodiment of figure 5, the transmit/receive coils 90 are placed under the table top 5 of the patient table 4, close to the top area where the head of the patient 20 is situated during an MRI examination. In this embodiment, the transmit/receive coils 90 will be moved together with the patient.

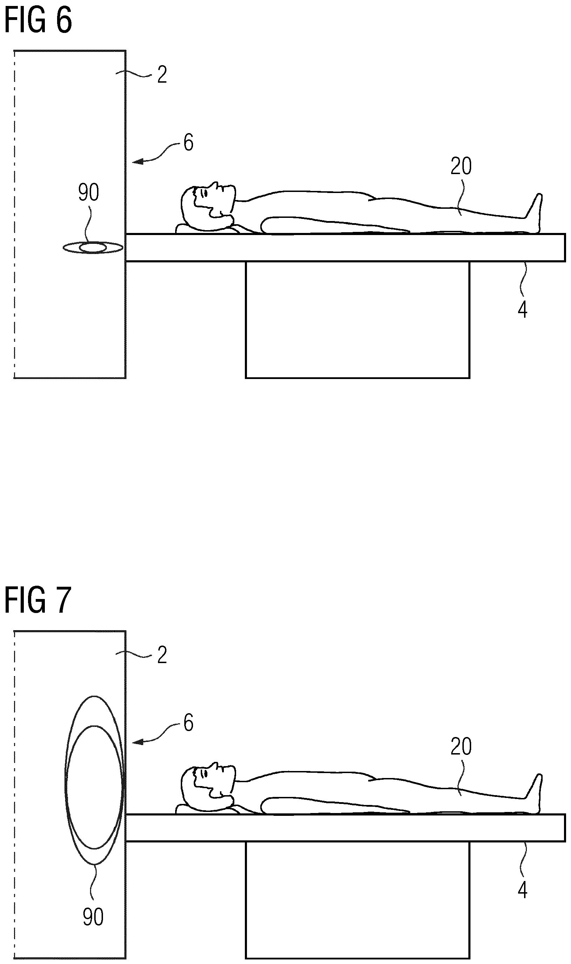

In the embodiment of figure 6, the transmit/receive coils 90 are integrated into the housing of the MRI scanner 2, inside the bore 6. Thereby, the patient 20 on the patient table 4 will be moved across the transmit/receive coils when the patient is being moved into the bore.

In the embodiment of figure 7, the transmit/receive coils 90 are arranged at the entrance of the bore 6, and are disposed circularly around the bore. Also in this embodiment, a metal detection is possible when the patient is being moved inside the bore 6 on the patient table 5, thereby allowing an early insight into the presence of metal objects in the patient.

## Claims

1. A Method for carrying out a dental magnetic resonance imaging examination of a field-of-view (22) within a subject, the field-of-view (22) including a dental area of the subject (20), with a magnetic resonance imaging system (1), the method comprising the following steps:
(a) determining whether a metallic object is present within or near the field-of-view,
(b) initiating a standard examination workflow (82) if it has been determined that no metallic object is present within or near the field-of-view, and initiating a modified examination workflow (80, 54, 60, 56, 62, 58, 64) that is different from the standard examination workflow if it has been determined that a metallic object is present within or near the field-of-view,
(c) carrying out the standard examination workflow (82) or the modified examination workflow depending on the initiation of step (b).

2. The method of claim 1, wherein the step of determining whether a metallic object is present within or near the field-of-view includes accessing (42) a dental record (30) of the subject and determining from the dental record whether a jaw and/or a tooth of the subject includes a metallic object, and optionally determining a position of said metallic object.

3. The method of claim 1 or 2, wherein the step of determining whether a metallic object is present within or near the field-of-view includes accessing (44) an image (32) of the mouth region of the subject, the image having been acquired with another imaging modality, in particular a photograph, X-ray image, CT-image or three-dimensional image, and analysing the image to determine whether a jaw and/or a tooth of the subject includes a metallic object, and optionally determining the position of said metallic object.

4. The method of one of the preceding claims, wherein the step of determining whether a metallic object is present includes carrying out an inductance measurement of the dental area of the subject using an inductance sensor (90) .

5. The method of one of the preceding claims, wherein the step of determining whether a metallic object is present includes a magnetic resonance measurement (46, 48).

6. The method of claim 5, wherein the magnetic resonance measurement comprises carrying out a spectroscopic measurement to acquire a magnetic resonance spectrum, wherein the magnetic resonance spectrum is analysed to determine off-resonant signal components as indication for the presence of metallic material.

7. The method of claim 5 or 6, wherein the magnetic resonance measurement includes a low-resolution magnetic resonance imaging scan (48), in particular having a spatial resolution which is higher in an occlusal plane than in a direction perpendicular thereto.

8. The method of one of claims 5 to 7, wherein the magnetic resonance measurement includes a diagnostic magnetic resonance imaging scan (60) with a first type of metal artefact correction activated, wherein the first type may also be no metal artefact correction.

9. The method of claim 8, wherein the image(s) acquired with the diagnostic magnetic resonance imaging scan are analysed to determine the presence of metallic objects, and wherein the modified examination workflow includes a diagnostic magnetic resonance imaging scan (62) with a second type of metal artefact correction activated, wherein the second type of metal artefact correction depends on a result of the analysis of the diagnostic magnetic resonance imaging scan with the first type of metal artefact correction.

10. The method of one of the preceding claims, wherein a presence of a metallic object is detected and the modified examination workflow is carried out, wherein the modified examination workflow includes determining a region within the field-of-view which is affected by the metallic object, and visualizing said affected region in a scout image used for planning the dental magnetic resonance imaging examination, and/or alerting the user when a diagnostic magnetic resonance imaging scan is planned in said affected region.

11. The method of one of the preceding claims, wherein a presence of a metallic object is detected and the modified examination workflow is carried out, and wherein the modified examination workflow includes determining a region within the field-of-view in which a magnetic resonance image includes a signal void caused by the metallic object, and modelling an acquired magnetic resonance image in this region on the basis of a jaw model, and/or augmenting an acquired magnetic resonance image in this region with image information taken from an image acquired with a non-MR imaging modality of the mouth of the subject.

12. The method of one of the preceding claims, wherein a presence of a metallic object is detected and the modified examination workflow is carried out, wherein the modified examination workflow includes alerting (80) a user that a dental magnetic resonance imaging examination will not yield acceptable image quality.

13. A non-transient computer-readable medium comprising a computer program with instructions that when carried out on a control unit (10) of a magnetic resonance imaging system (1) will cause the magnetic resonance imaging system (1) to carry out the method according to any one of the preceding claims.

14. A magnetic resonance imaging system (1) for examining a subject, in particular a patient, the magnetic resonance imaging system (1) comprising a magnetic resonance scanner (2) and a control unit (10),
wherein the control unit (10) is configured to
(a) determine whether a metallic object is present within or near the field-of-view, and
(b) initiate a standard examination workflow if it has been determined that no metallic object is present within or near the field-of-view, and initiate a modified examination workflow that is different from the standard examination workflow if it has been determined that a metallic object is present within or near the field-of-view,
and wherein the magnetic resonance scanner is configured to carry out the standard examination workflow or the modified examination workflow depending on the initiation of step (b).

15. The magnetic resonance imaging system (1) according to claim 14, wherein the magnetic resonance imaging system (1) further comprises an inductance sensor (90) configured for carrying out an inductance measurement of the dental area of the subject.
